# EUROPEAN PATENT SPECIFICATION

(11) **EP 0 658 543 B1**
(45) Date of publication and mention of the grant of the patent: **07.01.1998**
(21) Application number: 94919840.2
(22) Date of filing: 01.07.1994
(51) Int. Cl.: C07C 255/62, C07C 253/30, C07C 323/63, C07C 319/20

(54) **PROCESS FOR PRODUCING ALPHA-ALKOXYIMINOPHENYLACETONITRILE DERIVATIVE**
VERFAHREN ZUR HERSTELLUNG VON ALPHA-ALKOXY-IMINOPHENYLACETONITRIL
PROCEDE DE PREPARATION D'UN DERIVE DE ALPHA-ALCOXYIMINOPHENYLACETONITRILE

(30) Priority: 02.07.1993 JP 164710/93; 02.07.1993 JP 164711/93; 02.07.1993 JP 164712/93
(43) Date of publication of application: 21.06.1995
(73) Proprietor: SHIONOGI & CO., LTD., Osaka-shi, Osaka-fu 541 (JP); SUMITOMO CHEMICAL COMPANY LIMITED, Osaka-shi, Osaka 541 (JP)
(72) Inventor: TAKASE, Akira, Otsu-shi, Shiga 520-21 (JP); KAI, Hiroyuki, Yamatokoriyama-shi, Nara 639-11 (JP); MASUI, Moriyasu, Yokkaichi-shi, Mie 510-12 (JP); MASUMOTO, Katuhisa, Ibaraki-shi, Osaka 567 (JP); NAKAMURA, Akihiko, Takatsuki-shi, Osaka 567 (JP); KIYOSHIMA, Yujiro, Oita-shi, Oita 870-01 (JP); SASAKI, Mikio, Ibaraki-shi, Osaka 567 (JP)
(74) Representative: VOSSIUS & PARTNER
(86) International application number: JP9401077
(87) International publication number: WO9501330

(56) References cited:
- JP-A- 4 089 464
- JP-A- 6 219 986

## Description

The present invention relates to a process for producing α-alkoxyiminophenylacetonitrile derivatives of the general formula [III]: 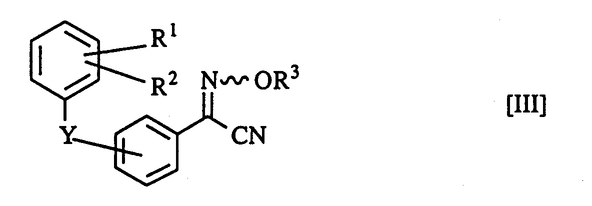 wherein R¹ and R² are the same or different and are independently C₁-C₅ alkyl, C₁-C₅ alkoxy, C₂-C₄ alkenyl, hydrogen, halogen or trifluoromethyl, R₃ is C₁-C₅ alkyl, and Y is -O-, -S-, -NCH₃-, -CH₂O-, -OCH₂-, -CH₂S- or -SCH₂-.

### Background Art

The alkoxyimino-containing compounds [III] are known as intermediates of medicaments or agricultural chemicals. As the process of their production, it is that hydroxyimino-containing compounds are reacted with an alkylating agent such as sulfuric ester, e.g., dimethyl sulfate in the presence of a base in an organic solvent. There is, however, a disadvantage that the reaction of an alkylating agent such as dimethyl sulfate in an organic solvent give rise to the formation of nitrone derivatives, as by-products, of the general formula [XII]: 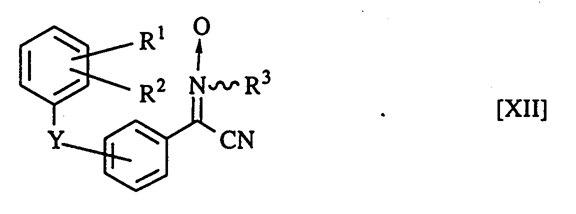 wherein R¹, R², R³ and Y are each as defined above, which causes a decrease in the yield and purity of the desired products.

### Disclosure of the Invention

The present inventors have extensively studied to develop a more favorable process for producing α-alkoxyiminophenylacetonitrile derivatives. As a result, they have found that the formation of nitrone derivatives as by-products can be reduced by effecting the reaction in a mixed solvent consisting of a hydrocarbon solvent and water in the presence of a phase transfer catalyst and the alkoxyimino-containing compounds [III] can therefore be produced in high yield, thereby completing the present invention.

Thus, the present invention provides a process for producing an α-alkoxyiminophenylacetonitrile derivative of the general formula [III]: 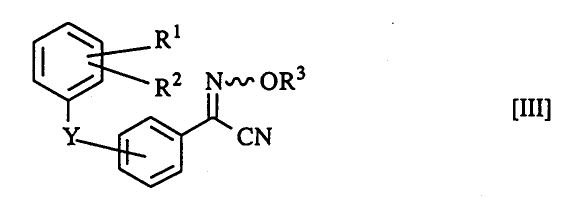 wherein R¹, R², R³ and Y are each as defined above, characterized in that an α-hydroxyiminophenylacetonitrile derivative of the general formula [ I ]: 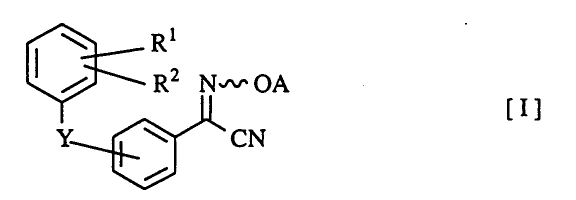 wherein R¹, R² and Y are each as defined above and A is hydrogen, an alkali metal or an alkaline earth metal, is reacted with a dialkyl sulfate of the general formula [II]:

(R³)₂SO₄ [II]

wherein R³ is as defined above in a mixed solvent consisting of a hydrocarbon solvent and water in the presence of a phase transfer catalyst.

The present invention will hereinafter be explained in detail.

As the R¹ and R² in the formula of α-hydroxyiminophenylacetonitrile derivative of the general formula [ I ], there can be mentioned, for example, C₁-C₅ alkyl such as methyl, ethyl, propyl, isopropyl, butyl, sec-butyl, tert-butyl, amyl and tert-amyl; and C₁-C₅ alkoxy such as methoxy, ethoxy, propoxy and butoxy; C₂-C₄ alkenyl such as ethenyl, propenyl and butenyl; hydrogen; halogen such as fluorine, chlorine and bromine; and trifluoromethyl. As the Y, there can be mentioned -O-, -S-, -NCH₃-, -CH₂O-, -OCH₂-, -CH₂S-, -SCH₂-, and the like. As the A, there can be mentioned, hydrogen; alkali metals such as sodium and potassium; and alkaline earth metals such as magnesium, barium and calcium, and the like.

As the specific example of the α-hydroxyiminophenylacetonitrile derivative, there can be mentioned, for example, α-hydroxyimino-2-(phenoxymethyl)phenylacetonitrile, α-hydroxyimino-2-(2-methylphenoxymethyl)phenylacetonitrile, α-hydroxyimino-2-(3-methylphenoxymethyl)phenylacetonitrile, α-hydroxyimino-2-(4-methylphenoxymethyl)phenylacetonitrile, α-hydroxyimino-3-(phenoxymethyl)phenylacetonitrile, α-hydroxyimino-3-(2-methylphenoxymethyl)phenylacetonitrile, α-hydroxyimino-3-(3-methylphenoxymethyl)phenylacetonitrile, α-hydroxyimino-3-(4-methylphenoxymethyl)phenylacetonitrile, α-hydroxyimino-4-(phenoxymethyl)phenylacetonitrile, α-hydroxyimino-4-(2-methylphenoxymethyl)phenylacetonitrile, α-hydroxyimino-4-(3-methylphenoxymethyl)phenylacetonitrile, α-hydroxyimino-4-(4-methylphenoxymethyl)phenylacetonitrile, α-hydroxyimino-2-(2-ethylphenoxymethyl)phenylacetonitrile, α-hydroxyimino-2-(3-ethylphenoxymethyl)phenylacetonitrile, α-hydroxyimino-2-(4-ethylphenoxymethyl)phenylacetonitrile, α-hydroxyimino-2-(2-propylphenoxymethyl)phenylacetonitrile, α-hydroxyimino-2-(4-propylphenoxymethyl)phenylacetonitrile, α-hydroxyimino-2-(2-isopropylphenoxymethyl)phenylacetonitrile, α-hydroxyimino-2-(3-isopropylphenoxymethyl)phenylacetonitrile, α-hydroxyimino-2-(4-isopropylphenoxymethyl)phenylacetonitrile, α-hydroxyimino-2-(2-sec-butylphenoxymethyl)phenylacetonitrile, α-hydroxyimino-2-(4-sec-butylphenoxymethyl)phenylacetonitrile, α-hydroxyimino-2-(2-tert-butylphenoxymethyl)phenylacetonitrile, α-hydroxyimino-2-(3-tert-butylphenoxymethyl)phenylacetonitrile, α-hydroxyimino-2-(4-tert-butylphenoxymethyl)phenylacetonitrile, α-hydroxyimino-2-(4-isopropyl-3-methylphenoxymethyl)phenylacetonitrile, α-hydroxyimino-2-(5-isopropyl-3-methylphenoxymethyl)phenylacetonitrile, α-hydroxyimino-2-(2-tert-butyl-5-methylphenoxymethyl)phenylacetonitrile, α-hydroxyimino-2-(4-tert-amylphenoxymethyl)phenylacetonitrile, α-hydroxyimino-2-(2,3-dimethylphenoxymethyl)phenylacetonitrile, α-hydroxyimino-2-(2,4-dimethylphenoxymethyl)phenylacetonitrile, α-hydroxyimino-2-(2,5-dimethylphenoxymethyl)phenylacetonitrile, α-hydroxyimino-2-(2,6-dimethylphenoxymethyl)phenylacetonitrile, α-hydroxyimino-2-(3,4-dimethylphenoxymethyl)phenylacetonitrile, α-hydroxyimino-2-(3,5-dimethylphenoxymethyl)phenylacetonitrile, α-hydroxyimino-2-(2,4-di-tert-butylphenoxymethyl)phenylacetonitrile, α-hydroxyimino-2-(3,5-di-tert-butylphenoxymethyl)phenylacetonitrile, α-hydroxyimino-2-(2,4-di-tert-amylphenoxymethyl) phenylacetonitrile, α-hydroxyimino-2-(2-methoxyphenoxymethyl)phenylacetonitrile, α-hydroxyimino-2-(4-butoxyphenoxymethyl)phenylacetonitrile, α-hydroxyimino-2-(2-methoxy-4-methylphenoxymethyl)phenylacetonitrile, α-hydroxyimino-2-(3,4-dimethoxyphenoxymethyl)phenylacetonitrile, α-hydroxyimino-2-(2-propenylphenoxymethyl)phenylacetonitrile, α-hydroxyimino-2-(2-allylphenoxymethyl)phenylacetonitrile, α-hydroxyimino-2-(2-allyl-6-methylphenoxymethyl)phenylacetonitrile, α-hydroxyimino-2-(2-fluorophenoxymethyl)phenylacetonitrile, α-hydroxyimino-2-(3-chlorophenoxymethyl)phenylacetonitrile, α-hydroxyimino-2-(4-chlorophenoxymethyl)phenylacetonitrile, α-hydroxyimino-2-(4-bromophenoxymethyl)phenylacetonitrile, α-hydroxyimino-2-(2,4-dichlorophenoxymethyl)phenylacetonitrile, α-hydroxyimino-2-(2,5-dichlorophenoxymethyl)phenylacetonitrile, α-hydroxyimino-2-(3-chloro-4-fluorophenoxymethyl)phenylacetonitrile, α-hydroxyimino-2-(4-fluoro-3-methylphenoxymethyl)phenylacetonitrile, α-hydroxyimino-2-(2-fluoro-6-methoxyphenoxymethyl)phenylacetonitrile, α-hydroxyimino-2-(2-chloro-4-methylphenoxymethyl)phenylacetonitrile, α-hydroxyimino-2-(2-chloro-5-methylphenoxymethyl)phenylacetonitrile, α-hydroxyimino-2-(4-chloro-2-methylphenoxymethyl)phenylacetonitrile, α-hydroxyimino-2-(4-chloro-3-methylphenoxymethyl)phenylacetonitrile, α-hydroxyimino-2-(2-trifluoromethylphenoxymethyl)phenylacetonitrile, α-hydroxyimino-2-(3-trifluoromethylphenoxymethyl)phenylacetonitrile, α-hydroxyimino-2-(4-trifluoromethylphenoxymethyl)phenylacetonitrile, α-hydroxyimino-2-(phenoxy)phenylacetonitrile, α-hydroxyimino-2-(phenylthio)phenylacetonitrile, α-hydroxyimino-2-(N-methylphenylamino)phenylacetonitrile, α-hydroxyimino-2-(2-methylbenzyloxy)phenylacetonitrile, α-hydroxyimino-2-(phenylthiomethyl)phenylacetonitrile, and the like.

As the R³ in the formula of the dialkyl sulfate of the general formula [II], there can be mentioned C₁-C₅ alkyl such as methyl, ethyl, propyl, butyl and amyl. As the typical example, there can be mentioned, for example, dimethyl sulfate, diethyl sulfate, and the like.

A feature of the present invention is in that the reaction of the α-hydroxyiminophenylacetonitrile derivative with the dialkyl sulfate is effected in a mixed solvent system consisting of a hydrocarbon solvent and water in the presence of a phase transfer catalyst. As the hydrocarbon solvent, there can be mentioned, for example, aromatic solvents such as benzene, toluene, xylene, chlorobenzene and o-dichlorobenzene; aliphatic solvents such as hexane and heptane, and the like. Preferably, aromatic solvents are used, and toluene is usually used. The amount of the solvent to be used is about 1 to 10 times the weight of the α-hydroxyiminophenylacetonitrile derivative. Also, water is usually used at such an amount that formed inorganic salts can be dissolved therein, and is not particularly limited.

As the phase transfer catalyst, there can be mentioned quaternary ammonium salts, quaternary phosphonium salts, and the like. Preferably, quaternary ammonium salts are used. As the quaternary ammonium salt, there can be mentioned, for example, tetra-n-butylammonium bromide, benzyltriethylammonium chloride, and the like.

In the case where a phase transfer catalyst is used, the amount to be used is usually 0.02 to 0.1 moles per mole of the α-hydroxyiminophenylacetonitrile derivative.

In the reaction, there is no particular limitation to the order or method for addition of the α-hydroxyiminophenylacetonitrile derivative as the starting material and the dialkyl sulfate. The maintenance of a higher ratio of the phase transfer catalyst to the α-hydroxyiminophenylacetonitrile derivative makes it possible to improve the yield and to reduce the formation of a nitrone compound as the by-product. Therefore, preferred are: (1) the method in which the phase transfer catalyst is added to a solvent containing the dialkyl sulfate, to which the α-hydroxyiminophenylacetonitrile derivative is then added dropwise or added in portions; (2) the method in which some part of the dialkyl sulfate is added to a solvent containing the phase transfer catalyst, and the α-hydroxyiminophenylacetonitrile derivative is then added dropwise or added in portions, during which the other part of the dialkyl sulfate is appropriately added in portions; (3) the method in which the dialkyl sulfate and the α-hydroxyiminophenylacetonitrile derivative are simultaneously added dropwise to a solvent containing the phase transfer catalyst; and the like.

To allow the reaction to proceed smoothly, an aqueous solution of hydroxides of alkali metals or alkaline earth metals can be used, and it is desirable to keep the pH of the reaction mixture from 9 to 14, preferably from 11 to 13.

The amount of the dialkyl sulfate to be used is usually 0.9 to 3 times, preferably 1.0 to 1.5 times, to one mole of the α-hydroxyiminophenylacetonitrile derivative.

The reaction temperature is usually 0 to 60°C, preferably 10 to 40°C, and the reaction time is usually about 0.5 to 20 hours.

The α-alkoxyiminophenylacetonitrile derivative formed can readily be isolated by subjecting the reaction mixture to phase separation to remove the aqueous layer, washing the organic layer with a base such as an aqueous sodium hydroxide solution, and then concentrating the organic layer under reduced pressure, followed by drying.

If necessary, purification can also be employed by an ordinary purification technique such as column chromatography.

The R¹, R² and Y in the formula of the α-alkoxyiminophenylacetonitrile derivative of the general formula [III] are each as defined above. As the specific example, there can be mentioned, for example, α-methoxyimino-2-(phenoxymethyl)phenylacetonitrile, α-methoxyimino-2-(2-methylphenoxymethyl)phenylacetonitrile, α-methoxyimino-2-(3-methylphenoxymethyl)phenylacetonitrile, α-methoxyimino-2-(4-methylphenoxymethyl)phenylacetonitrile, α-methoxyimino-3-(phenoxymethyl)phenylacetonitrile, α-methoxyimino-3-(2-methylphenoxymethyl)phenylacetonitrile, α-methoxyimino-3-(3-methylphenoxymethyl)phenylacetonitrile, α-methoxyimino-3-(4-methylphenoxymethyl)phenylacetonitrile, α-methoxyimino-4-(phenoxymethyl)phenylacetonitrile, α-methoxyimino-4-(2-methylphenoxymethyl)phenylacetonitrile, α-methoxyimino-4-(3-methylphenoxymethyl)phenylacetonitrile, α-methoxyimino-4-(4-methylphenoxymethyl)phenylacetonitrile, α-methoxyimino-2-(2-ethylphenoxymethyl)phenylacetonitrile, α-methoxyimino-2-(3-ethylphenoxymethyl)phenylacetonitrile, α-methoxyimino-2-(4-ethylphenoxymethyl)phenylacetonitrile, α-methoxyimino-2-(2-propylphenoxymethyl)phenylacetonitrile, α-methoxyimino-2-(4-propylphenoxymethyl)phenylacetonitrile, α-methoxyimino-2-(2-isopropylphenoxymethyl)phenylacetonitrile, α-methoxyimino-2-(3-isopropylphenoxymethyl)phenylacetonitrile, α-methoxyimino-2-(4-isopropylphenoxymethyl)phenylacetonitrile, α-methoxyimino-2-(2-sec-butylphenoxymethyl)phenylacetonitrile, α-methoxyimino-2-(4-sec-butylphenoxymethyl)phenylacetonitrile, α-methoxyimino-2-(2-tert-butylphenoxymethyl)phenylacetonitrile, α-methoxyimino-2-(3-tert-butylphenoxymethyl)phenylacetonitrile, α-methoxyimino-2-(4-tert-butylphenoxymethyl)phenylacetonitrile α-methoxyimino-2-(4-isopropyl-3-methylphenoxymethyl)phenylacetonitrile, α-methoxyimino-2-(5-isopropyl-3-methylphenoxymethyl)phenylacetonitrile, α-methoxyimino-2-(2-tert-butyl-5-methylphenoxymethyl)phenylacetonitrile, α-methoxyimino-2-(4-tert-amylphenoxymethyl)phenylacetonitrile, α-methoxyimino-2-(2,3-dimethylphenoxymethyl)phenylacetonitrile, α-methoxyimino-2-(2,4-dimethylphenoxymethyl)phenylacetonitrile, α-methoxyimino-2-(2,5-dimethylphenoxymethyl)phenylacetonitrile, α-methoxyimino-2-(2,6-dimethylphenoxymethyl)phenylacetonitrile, α-methoxyimino-2-(3,4-dimethylphenoxymethyl)phenylacetonitrile, α-methoxyimino-2-(3,5-dimethylphenoxymethyl)phenylacetonitrile, α-methoxyimino-2-(2,4-di-tert-butylphenoxymethyl)phenylacetonitrile, α-methoxyimino-2-(3,5-di-tert-butylphenoxymethyl)phenylacetonitrile, α-methoxyimino-2-(2,4-di-tert-amylphenoxymethyl)phenylacetonitrile, α-methoxyimino-2-(2-methoxyphenoxymethyl)phenylacetonitrile, α-methoxyimino-2-(4-butoxyphenoxymethyl)phenylacetonitrile, α-methoxyimino-2-(2-methoxy-4-methylphenoxymethyl)phenylacetonitrile, α-methoxyimino-2-(3,4-dimethoxyphenoxymethyl)phenylacetonitrile, α-methoxyimino-2-(2-propenylphenoxymethyl)phenylacetonitrile, α-methoxyimino-2-(2-allylphenoxymethyl)phenylacetonitrile, α-methoxyimino-2-(2-allyl-6-methylphenoxymethyl)phenylacetonitrile, α-methoxyimino-2-(2-fluorophenoxymethyl)phenylacetonitrile, α-methoxyimino-2-(3-chlorophenoxymethyl)phenylacetonitrile, α-methoxyimino-2-(4-chlorophenoxymethyl)phenylacetonitrile, α-methoxyimino-2-(4-bromophenoxymethyl)phenylacetonitrile, α-methoxyimino-2-(2,4-dichlorophenoxymethyl)phenylacetonitrile, α-methoxyimino-2-(2,5-dichlorophenoxymethyl)phenylacetonitrile, α-methoxyimino-2-(3-chloro-4-fluorophenoxymethyl)phenylacetonitrile, α-methoxyimino-2-(4-fluoro-3-methylphenoxymethyl)phenylacetonitrile, α-methoxyimino-2-(2-fluoro-6-methoxyphenoxymethyl)phenylacetonitrile, α-methoxyimino-2-(2-chloro-4-methylphenoxymethyl)phenylacetonitrile α-methoxyimino-2-(2-chloro-5-methylphenoxymethyl)phenylacetonitrile, α-methoxyimino-2-(4-chloro-2-methylphenoxymethyl)phenylacetonitrile, α-methoxyimino-2-(4-chloro-3-methylphenoxymethyl)phenylacetonitrile, α-methoxyimino-2-(2-trifluoromethylphenoxymethyl)phenylacetonitrile, α-methoxyimino-2-(3-trifluoromethylphenoxymethyl)phenylacetonitrile, α-methoxyimino-2-(4-trifluoromethylphenoxymethyl)phenylacetonitrile, α-methoxyimino-2-(phenoxy)phenylacetonitrile, α-methoxyimino-2-(phenythio)phenylacetonitrile, α-methoxyimino-2-(N-methylphenylamino)phenylacetonitrile, α-methoxyimino-2-(2-methylbenzyloxy)phenylacetonitrile, α-methoxyimino-2-(phenylthiomethyl)phenylacetonitrile, α-ethoxyimino-2-(phenoxymethyl)phenylacetonitrile, α-ethoxyimino-2-(2-methylphenoxymethyl)phenylacetonitrile, α-ethoxyimino-2-(2,5-dimethylphenoxymethyl)phenylacetonitrile, and the like.

The α-hydroxyiminophenylacetonitrile derivative [ I ] to be used in the present invention can be produced, for example, by the method as described below.

The α-hydroxyiminophenylacetonitrile derivative of the general formula [ I ]: 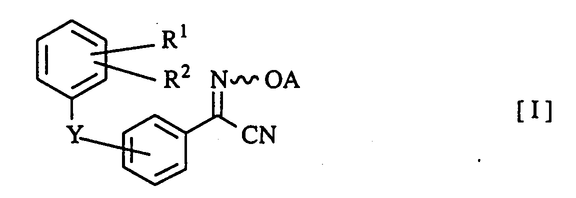 wherein R¹, R², Y and A are each as defined above, can be produced by reacting a substituted phenylacetonitrile derivative of the general formula [ IV]: 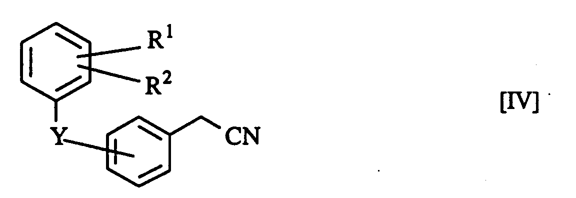 wherein R¹, R² and Y are each as defined above, with an alkyl nitrite of the general formula [V]:

R⁴-ONO [V]

wherein R⁴ is C₁-C₆ alkyl, in a mixed solvent consisting of an aromatic solvent and a lower alcohol in the presence of a base.

As the R¹, R² and Y in the formula of the substituted phenylacetonitrile derivative of the general formula [IV], there can be mentioned those which are as defined above.

As the specific example of the substituted phenylacetonitrile derivative, there can be mentioned 2-(phenoxymethyl)phenylacetonitrile 2-(2-methylphenoxymethyl)phenylacetonitrile, 2-(3-methylphenoxymethyl)phenylacetonitrile, 2-(4-methylphenoxymethyl)phenylacetonitrile, 3-(phenoxymethyl)phenylacetonitrile, 3-(2-methylphenoxymethyl)phenylacetonitrile, 3-(3-methylphenoxymethyl)phenylacetonitrile, 3-(4-methylphenoxymethyl)phenylacetonitrile, 4-(phenoxymethyl)phenylacetonitrile, 4-(2-methylphenoxymethyl)phenylacetonitrile, 4-(3-methylphenoxymethyl)phenylacetonitrile 4-(4-methylphenoxymethyl)phenylacetonitrile, 2-(2-ethylphenoxymethyl)phenylacetonitrile, 2-(3-ethylphenoxymethyl)phenylacetonitrile, 2-(4-ethylphenoxymethyl)phenylacetonitrile, 2-(2-propylphenoxymethyl)phenylacetonitrile, 2-(4-propylphenoxymethyl)phenylacetonitrile, 2-(2-isopropylphenoxymethyl)phenylacetonitrile, 2-(3-isopropylphenoxymethyl)phenylacetonitrile, 2-(4-isopropylphenoxymethyl)phenylacetonitrile, 2-(2-sec-butylphenoxymethyl)phenylacetonitrile, 2-(4-sec-butylphenoxymethyl)phenylacetonitrile 2-(2-tert-butylphenoxymethyl)phenylacetonitrile, 2-(3-tert-butylphenoxymethyl)phenylacetonitrile, 2-(4-tert-butylphenoxymethyl)phenylacetonitrile, 2-(4-isopropyl-3-methylphenoxymethyl)phenylacetonitrile, 2-(5-isopropyl-3-methylphenoxymethyl)phenylacetonitrile, 2-(2-tert-butyl-5-methylphenoxymethyl)phenylacetonitrile, 2-(4-tert-amylphenoxymethyl)phenylacetonitrile, 2-(2,3-dimethylphenoxymethyl)phenylacetonitrile 2-(2,4-dimethylphenoxymethyl)phenylacetonitrile, 2-(2,5-dimethylphenoxymethyl)phenylacetonitrile, 2-(2,6-dimethylphenoxymethyl)phenylacetonitrile, 2-(3,4-dimethylphenoxymethyl)phenylacetonitrile, 2-(3,5-dimethylphenoxymethyl)phenylacetonitrile, 2-(2,4-di-tert-butylphenoxymethyl)phenylacetonitrile, 2-(3,5-di-tert-butylphenoxymethyl)phenylacetonitrile, 2-(2,4-di-tert-amylphenoxymethyl)phenylacetonitrile, 2-(2-methoxyphenoxymethyl)phenylacetonitrile, 2-(4-butoxyphenoxymethyl)phenylacetonitrile, 2-(2-methoxy-4-methylphenoxymethyl)phenylacetonitrile, 2-(3,4-dimethoxyphenoxymethyl)phenylacetonitrile, 2-(2-propenylphenoxymethyl)phenylacetonitrile, 2-(2-allylphenoxymethyl)phenylacetonitrile, 2-(2-allyl-6-methylphenoxymethyl)phenylacetonitrile, 2-(2-fluorophenoxymethyl)phenylacetonitrile 2-(3-chlorophenoxymethyl)phenylacetonitrile 2-(4-chlorophenoxymethyl)phenylacetonitrile, 2-(4-bromophenoxymethyl)phenylacetonitrile, 2-(2,4-dichlorophenoxymethyl)phenylacetonitrile, 2-(2,5-dichlorophenoxymethyl)phenylacetonitrile, 2-(3-chloro-4-fluorophenoxymethyl)phenylacetonitrile, 2-(4-fluoro-3-methylphenoxymethyl)phenylacetonitrile, 2-(2-fluoro-6-methoxyphenoxymethyl)phenylacetonitrile, 2-(2-chloro-4-methylphenoxymethyl)phenylacetonitrile, 2-(2-chloro-5-methylphenoxymethyl)phenylacetonitrile, 2-(4-chloro-2-methylphenoxymethyl)phenylacetonitrile, 2-(4-chloro-3-methylphenoxymethyl)phenylacetonitrile, 2-(2-trifluoromethylphenoxymethyl)phenylacetonitrile 2-(3-trifluoromethylphenoxymethyl)phenylacetonitrile 2-(4-trifluoromethylphenoxymethyl)phenylacetonitrile, 2-(phenoxy)phenylacetonitrile, 2-(phenylthio)phenylacetonitrile, 2-(N-methylphenylamino)phenylacetonitrile 2-(2-methylbenzyloxy)phenylacetonitrile, 2-(phenylthiomethyl)phenylacetonitrile, and the like.

The substituted phenylacetonitrile [IV] may contain, for example, the starting materials and by-products in its production as the impurities.

As such impurities, for example, in the case where the substituted phenylacetonitrile [IV] is produced through the following route (1), there can be mentioned, for example, toluene derivative [IX], benzyl halide derivative [VIII], benzal halide derivative [X], and the like.

### (Route 1)

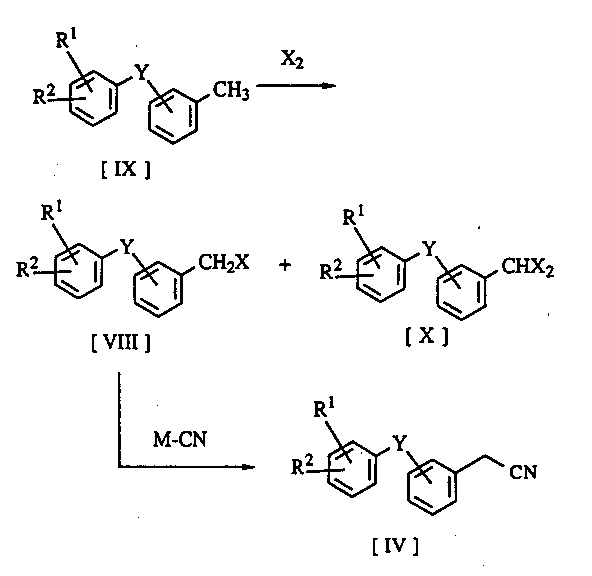

Also, in the case where the substituted phenylacetonitrile [IV] is produced through the following route (2), there can be mentioned as the impurities, benzyl halide derivative [VIII'], α,α'-disubstituted xylene derivative [XI], and the like, as well as α,α'-dihaloxylene derivative [VI], and the starting materials and by-products in its production.

### (Route 2)

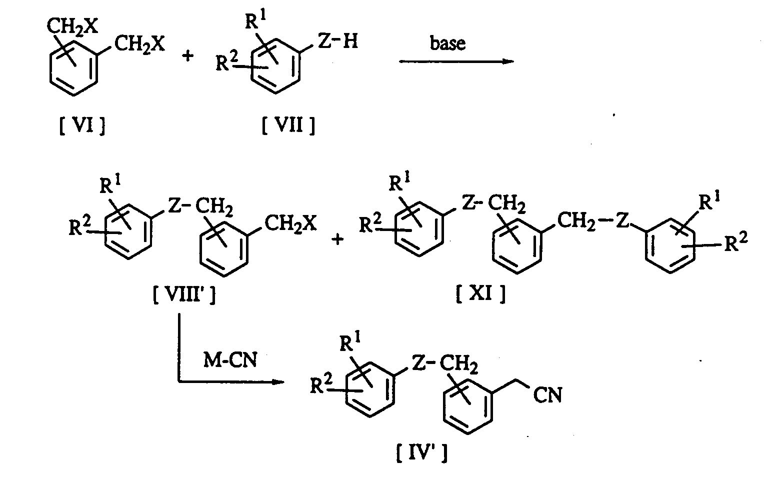

In the reaction of the substituted phenylacetonitrile [IV] with the alkyl nitrite, there can be mentioned as the alkyl nitrite of the general formula [V] to be used, for example, methyl nitrite, ethyl nitrite, n-propyl nitrite, isopropyl nitrite, butyl nitrite, amyl nitrite, isoamyl nitrite, hexyl nitrite, and the like. Such an alkyl nitrite may be used after synthesized by the known methods or may be obtained from commercial sources.

The reaction of the substituted phenylacetonitrile derivative with the alkyl nitrite is effected in a solvent in the presence of a base.

The amount of the alkyl nitrite to be used is usually 0.8 to 10 moles, preferably 1 to 2 moles, to 1 mole of the substituted phenylacetonitrile derivative.

The reaction is usually effected in the presence of a base. As the base to be used, there can be mentioned, for example, inorganic bases such as hydroxides of alkali metals, e.g., sodium hydroxide and potassium hydroxide; hydroxides of alkaline earth metals, e.g., calcium hydroxide; and carbonates of alkali metals, e.g., sodium carbonate and potassium carbonate; and organic bases can also be used, such as alkoxides of alkali metals, e.g., sodium ethoxide. Two or more kinds of these bases can be used in combination.

The amount of the base to be used is usually 0.8 to 10 equivalents, preferably 1 to 2 equivalents, to the substituted phenylacetonitrile derivative.

As the solvent, a combination of an aromatic solvent and a lower alcohol is usually used; there can be mentioned, for example, aromatic solvents such as benzene, toluene, xylene, chlorobenzene, o-dichlorobenzene, and the like. Particularly preferred is toluene. As the lower alcohol, there can be mentioned, for example, methyl alcohol, ethyl alcohol, propyl alcohol, isopropyl alcohol, butyl alcohol, amyl alcohol, isoamyl alcohol, hexyl alcohol, and the like. Particularly preferred is methyl alcohol or a lower alcohol having the same alkyl group as that of the alkyl nitrite. The ratio of the lower alcohol to the aromatic solvent is such that the weight of the lower alcohol is usually 0.01 to 0.5 times, preferably 0.03 to 0.2 times, the weight of the aromatic solvent.

The amount of the solvent to be used is 1 to 10 times, preferably 2 to 5 times, the weight of the substituted phenylacetonitrile derivative.

The reaction temperature is 0 to 60°C, preferably 10 to 40°C. The reaction time is usually about 0.5 to 20 hours.

Thus, the reaction mixture containing the α-hydroxyiminophenylacetonitrile [ I ] is obtained, and this mixture can be extracted with water to separate the desired product [ I ] in the aqueous layer from the impurities in the organic layer. Therefore, to obtain the α-hydroxyiminophenylacetonitrile [ I ] in high purity, it is preferred to extract the reaction mixture with water.

In the case of water extraction, water is added to the reaction mixture. When a hydrophilic organic solvent is contained in the reaction mixture, the reaction mixture is usually used after the removal of the hydrophilic organic solvent by a technique such as concentration.

Water is added so that the total amount thereof becomes usually about 1 to 20 times the weight of the crude phenylacetonitrile used.

Further, the addition of a water-insoluble or sparingly water-soluble organic solvent is also preferable; for example, aliphatic hydrocarbons such as hexane and heptane; aromatic hydrocarbons such as benzene, toluene and xylene; ethers such as diethyl ether; ketones such as methyl isobutyl ketone; halogenated hydrocarbons such as dichloromethane, 1,2-dichloroethane, chlorobenzene and o-dichlorobenzene, and the like. The addition of such an organic solvent makes it possible to separate the impurities with high efficiency.

In that case, the organic solvent is added so that the total amount thereof becomes usually about 0.1 to 5 times the weight of water added.

The aqueous layer separated by the extraction procedure can be washed with a water-insoluble or sparingly water-soluble organic solvent.

Then, the separated aqueous layer is neutralized with an acid, but it can also be supplied as the starting material in the subsequent step without conducting neutralization. As the acid when the acid is used, either an organic acid or an inorganic acid may be used, and preferred is an inorganic acid. As the preferred inorganic acid, there can be mentioned, for example, mineral acids such as hydrochloric acid, sulfuric acid and nitric acid.

The acid is used until the pH of the system becomes 7 or lower, preferably 4 or lower, and more preferably 2 or lower.

The neutralization with an acid is usually effected at -10 to 100°C, preferably 0 to 40°C.

Thus, the α-hydroxyiminophenylacetonitrile [ I ] is obtained, and the α-hydroxyiminophenylacetonitrile [ I ] can be isolated by the ordinary methods. For example, the isolation can be achieved by filtration when the α-hydroxyiminophenylacetonitrile [ I ] is obtained as crystals, or for example, by extraction with an organic solvent, followed by removal of the organic solvent by distillation, when obtained in an oily product.

Further, from the organic layer obtained by the phase separation, the aromatic solvent and the lower alcohol can readily be separated and recovered by distillation.

Next, the following will describe the process for producing the substituted phenylacetonitrile [IV].

The substituted phenylacetonitrile [IV] to be used in the present invention, although it is not particularly limited, can be produced, for example, by the above-described route (1), (2) and the like.

In the case where the substituted phenylacetonitrile [IV] is produced through the route (I), the substituted phenylacetonitrile [IV] can be produced, for example, by reacting the toluene derivative [IX] with halogen to produce the benzyl halide derivative [VIII] and then reacting this derivative with a cyano compound.

As the substituents R¹ and R² in the toluene derivative [IX], there can be mentioned, for example, those which are as defined above. As the Y, there can be mentioned -O-, -S-, -NCH₃-, -OCH₂-, -CH₂O-, -SCH₂-, -CH₂S-, and the like.

As the specific compound, there can be mentioned, for example, 1-methyl-2-phenoxybenzene, 1-(2-fluorophenoxy)-2-methylbenzene, 1-(4-bromophenoxy)-3-methylbenzene, 1-(2-methoxyphenoxy)-2-methylbenzene, 1-(2-isopropoxyphenoxy)-4-methylbenzene, 1-(4-butoxyphenoxy)-4-methylbenzene, 1-(2,3-dimethoxyphenoxy)-2-methylbenzene, 1-methyl-2-(phenylthio)benzene, 1-(2-methoxyphenylthio)-3-methylbenzene, and the like.

The benzyl halide derivative [VIII] can also be produced from the toluene derivative [IX], for example, in accordance with the methods as described in Japanese Patent Laid-open Publication Nos. 56-166142 and 57-18644.

In the reaction of the benzyl halide derivative [VIII] with the cyano compound,the benzyl halide derivative [VIII] to be used may be a crude benzyl halide derivative containing impurities such as the toluene derivative [IX] and the benzal halide derivative [X], or of course, it may have high purity.

As the cyano compound, there can be mentioned, for example, sodium cyanide, potassium cyanide, or mixtures thereof. It can also be used after prepared by reacting hydrogen cyanide with an alkali metal salt such as sodium hydroxide, potassium hydroxide, sodium carbonate and potassium carbonate in the reaction system.

The amount of such a cyano compound to be used is usually 0.8 to 10 times, preferably 1 to 2 times, the moles of the benzyl halide derivative [VIII].

The reaction is usually effected in the presence of a solvent. As such a solvent, there can be mentioned, for example, aprotic polar solvents such as dimethylformamide and dimethylsulfoxide. In the presence of a phase transfer catalyst, water and a water-insoluble or sparingly water-soluble organic solvent can also be used. As such an organic solvent, there can be mentioned, for example, aliphatic hydrocarbons such as hexane and heptane; aromatic hydrocarbons such as benzene, toluene and xylene; halogenated hydrocarbons such as dichloromethane, 1,2-dichloroethane, chlorobenzene and o-dichlorobenzene; and mixtures thereof. The solvent is used at an amount which is usually 1 to 10 times the weight of the benzyl halide derivative used.

As the phase transfer catalyst, there can be mentioned quaternary ammonium salts, quaternary phosphonium salts, and the like. Preferred are quaternary ammonium salts. As the quaternary ammonium salt, there can be usually used, for example, tetra-n-butylammonium bromide, benzyltriethylammonium chloride, or the like.

The amount of the phase transfer catalyst to be used is usually 0.001 to 1 time, preferably 0.005 to 0.1 times, the moles of the benzyl halide derivative [VIII].

The reaction is usually effected at a temperature of from 0°C to the refluxing temperature of the solvent.

Thus, the cyano compound is selectively reacted with the benzyl halide derivative [VIII] to form the substituted phenylacetonitrile [IV], while the toluene derivative [IX] and the benzal halide derivative [X] are kept in substantially quantitative manner.

The reaction mixture obtained can be subjected to the production of the starting material of the present invention by the ordinary methods, for example, after washed with water, followed by removal of the solvent by distillation, or further after the toluene derivative [IX] and the like are removed by distillation.

Also, in the case where the substituted phenylacetonitrile [IV] is produced through the route (2), the crude nitrile containing the phenylacetonitrile [IV'] can be produced, for example, by reacting the α,α'-dihaloxylene derivative [VI] with the phenol derivative [VII] in the presence of a base to produce the benzyl halide derivative [VIII'] and then reacting this derivative with a cyano compound.

As the specific compound of the α,α'-dihaloxylene derivative [VI], there can be mentioned, for example, α,α'-dichloro-o-xylene, α,α'-dichloro-m-xylene, α,α'-dichloro-p-xylene, α,α'-dibromo-o-xylene, α,α'-dibromo-m-xylene, α,α'-dibromo-p-xylene, and the like.

As the substituents R¹ and R² in the phenol derivative [VII], there can be mentioned, for example, those which are as defined above. As the Z, there can be mentioned O, S, and the like. As the specific compound, there can be mentioned, for example, phenol, 2-fluorophenol, 3-chlorophenol, 4-chlorophenol, 4-bromophenol, 3-trifluoromethylphenol, 2-methylphenol, 4-i-propylphenol, 3-t-butylphenol, 2-methoxyphenol, 4-butoxyphenol, 2,4-dichlorophenol, 2,5-dichlorophenol, 3-chloro-4-fluorophenol, 4-fluoro-3-methylphenol, 4-chloro-2-methylphenol, 2-fluoro-6-methoxyphenol, 2,4-dimethylphenol, 2,5-dimethylphenol, 2-methoxy-4-methylphenol, 3,4-dimethoxyphenol, thiophenol, 4-fluorothiophenol, 2-chlorothiophenol, 3-bromothiophenol, 2-methoxythiophenol, 2,5-dichlorothiophenol, 3,4-dichlorothiophenol, 2,4-dimethylthiophenol, 2,5-dimethylthiophenol, 3,5-dimethylthiophenol, 3-trifluoromethylphenol, and the like.

The α,α'-dihaloxylene derivative [VI] is used at an amount which is usually not less than 1.5 times, preferably 2 to 6 times, the moles of the phenol derivative [VII].

As the base, there can be mentioned, for example, hydroxides or carbonates of alkali metals or alkaline earth metals, such as sodium hydroxide, potassium hydroxide, magnesium hydroxide, calcium hydroxide, barium hydroxide, sodium carbonate and potassium carbonate. The amount to be used is usually 0.7 to 1.5 times, preferably 0.9 to 1.2 moles, the moles of the phenol derivative [VII].

The reaction is usually effected by adding dropwise a base to a mixture of the solvent, α,α'-dihaloxylene derivative [VI], phenol derivative [VII], and the like.

As the solvent, there can be usually used water or a mixed solvent of water and an organic solvent. As such an organic solvent, there can be mentioned, for example, aromatic hydrocarbons such as toluene and xylene; aliphatic hydrocarbons such as hexane, cyclohexane and heptane; halogenated hydrocarbons such as chlorobenzene, dichlorobenzene, chloroform and 1,2-dichloroethane; ketones such as acetone and methyl isobutyl ketone; ethers such as diisopropyl ether and methyl-t-butyl ether, and the like.

The amount of the solvent to be used is usually about 1 to 20 times the weight of the phenol derivative [VII].

Also, when the reaction is effected in the presence of a two-layer solvent system consisting of an organic solvent and water, it is also effective to allow a phase transfer catalyst to coexist therewith. As the phase transfer catalyst, there can be mentioned, for example, quaternary ammonium salts such as tetra-n-butylammonium bromide and benzyltriethylammonium chloride. The amount to be used is usually 0.02 to 0.1 times the moles of the phenol derivative [VII].

The reaction is usually effected at about 20 to 100°C. The benzyl halide derivative [VIII'] formed can be isolated by a technique such as extraction, distillation or recrystallization.

Then, the crude nitrile containing the substituted phenylacetonitrile [IV] can be produced by reacting the benzyl halide derivative [VIII'] with the cyano compound, and the benzyl halide derivative [VIII'] to be used may be a crude benzyl halide derivative containing impurities such as α,α'-disubstituted xylene derivative [XI] and the starting materials and by-products in the production of α,α'-dihaloxylene derivative [VI], or of course, it may have high purity.

The cyanation and post-treatment are effected under the same conditions as in the above route (1). As the substituted phenylacetonitrile [IV] obtained, there can be mentioned, for example, those where the substituents R¹ and R² are each as defined above.

### Examples

The present invention will be further illustrated by the following Examples, but it is needless to say that the present invention is not limited to the Examples.

### Reference Example 1-(1) Production of benzyl halide

To 1619 g of toluene were added 788 g (4.50 mol) of α,α'-dichloro-o-xylene, 183 g (1.50 mol) of 2,5-dimethylphenol and 24.2 g (0.075 mol) of tetra-n-butylammonium bromide with stirring, to which 1246 g of water was added, and the mixture was heated to 60°C.

To this mixture was added dropwise 244 g (1.65 mol) of 27% aqueous sodium hydroxide solution over 5 hours, and the mixture was kept at the same temperature for 3 hours and then cooled to room temperature. The aqueous layer was removed by phase separation, whereas the organic layer was washed with 810 g of 5% hydrochloric acid and washed twice with 810 g of water, and the α,α'-dichloro-o-xylene was removed by distillation, which afforded 364 g of a mixture containing 279 g (1.07 mol, 71.3% yield) of 2-(2,5-dimethylphenoxymethyl)benzyl chloride and 57.7 g (0.167 mol, 22.2% yield) of 1,2-bis(2,5-dimethylphenoxymethyl)benzene. The mixture thus obtained can be used in the subsequent step after diluted by addition of toluene.

### Reference Example 1-(2)

To 140.0 g of toluene were added 113.8 g (0.65 mol) of α,α'-dichloro-o-xylene, 15.9 g (0.13 mol) of 2,5-dimethylphenol and 2.10 g (0.0065 mol) of tetra-n-butylammonium bromide with stirring, to which 108.0 g of water was added, and the mixture was heated to 60°C.

To this mixture was added dropwise 21.2 g (0.143 mol) of 27% aqueous sodium hydroxide solution over 5 hours, and the mixture was kept at the same temperature for 2 hours and then cooled to room temperature. The aqueous layer was removed by phase separation, whereas the organic layer was washed with 70.2 g of 5% hydrochloric acid and washed twice with 70.2 g of water, and the α,α'-dichloro-o-xylene was removed by distillation, which afforded 33.5 g of a mixture containing 26.4 g (0.101 mol, 77.8% yield) of 2-(2,5-dimethylphenoxymethyl)benzyl chloride and 3.08 g (0.0089 mol, 13.7% yield) of 1,2-bis(2,5-dimethylphenoxymethyl)benzene. The mixture thus obtained can be used in the subsequent step after diluted by addition of toluene.

### Reference Example 1-(3)

The reaction and post-treatment were conducted in the same manner as in Reference Example 1-(2), except that 45.5 g (0.26 mol) of α,α'-dichloro-o-xylene was used, which afforded 36.7 g of a mixture containing 20.7 g (0.079 mol, 61.1% yield) of 2-(2,5-dimethylphenoxymethyl)benzyl chloride and 6.97 g (0.020 mol, 30.9% yield) of 1,2-bis(2,5-dimethylphenoxymethyl)benzene.

### Reference Example 1-(4)

To 22.8 g (0.13 mol) of α,α'-dichloro-o-xylene, 15.9 g (0.13 mol) of 2,5-dimethylphenol and 2.10 g (0.0065 mol) of tetra-n-butylammonium bromide were added 140.3 g of toluene and then 108.0 g of water, followed by stirring. To this mixture was added dropwise 21.2 g (0.143 mol) of 27% aqueous sodium hydroxide solution at 60°C over 5 hours, and the mixture was further kept at 60°C for 9 hours. The reaction mixture was cooled to room temperature, and the aqueous layer was removed by phase separation. The organic layer was washed with 70.2 g of 5% hydrochloric acid and then washed twice with 70.2 g of water. The organic layer was concentrated to give 25.7 g of a mixture containing 12.1 g (0.0464 mol, 35.7% yield) of 2-(2,5-dimethylphenoxymethyl)benzyl chloride and 11.7 g (0.0338 mol, 52.0% yield) of 1,2-bis(2,5-dimethylphenoxymethyl)benzene.

### Reference Example 1-(5)

This Example was conducted in accordance with Reference Example 1-(1), except that 214 g (1.50 mol) of 4-chloro-2-methylphenol was used instead of 183 g (1.50 mol) of 2,5-dimethylphenol and the temperature was kept at 60°C for 3 hours in Reference Example 1-(1). Thus, 381 g of a solution containing 2-(4-chloro-2-methylphenoxymethyl)benzyl chloride was obtained, which was analyzed by gas chromatography, and it was found that the content of 2-(4-chloro-2-methylphenoxymethyl)benzyl chloride was 72.0%. The yield of 2-(4-chloro-2-methylphenoxymethyl)benzyl chloride was 65.0% on the basis of 4-chloro-2-methylphenol.

Then, 117 g of 2-(4-chloro-2-methylphenoxymethyl)benzyl chloride was distilled under reduced pressure to give 58.9 g of the fraction at a boiling point of 162 to 176°C (0.4 to 0.6 mmHg). As a result of the analysis by gas chromatography, the purity of 2-(4-chloro-2-methylphenoxymethyl)benzyl chloride was 93.2%.

### Reference example 2-(1) Production of phenylacetonitrile

A mixture comprising 266 g of water, 66.6 g (1.36 mol) of sodium cyanide, 16.4 g (0.051 mol) of tetra-n-butylammonium bromide and 439 g of toluene was heated to 80°C with stirring, to which 715 g of a toluene solution of a mixture containing 266 g (1.02 mol) of 2-(2,5-dimethylphenoxymethyl)benzyl chloride and 56.5 g (0.163 mol) of 1,2-bis(2,5-dimethylphenoxymethyl)benzene, which had been produced in accordance with Reference Example 1-(2), was added dropwise over 5 hours, and the mixture was further kept at the same temperature for 3 hours.

After cooling to room temperature, the aqueous layer was removed by phase separation, and the organic layer was washed three times with 379 g of 1% aqueous sodium hydroxide solution, and washed with 379 g of water and then with 379 g of 10% saline solution, which afforded 1141 g of the organic layer. To 716 g of this organic layer was added 43.3 g (0.064 mol) of 11% aqueous sodium hypochlorite solution, and the mixture was stirred at 23°C for 3 hours, after which the precipitated insoluble materials were removed by filtration. The organic layer was washed with 100 g of 10% aqueous sodium sulfite solution, with 100 g of water and then with 10% saline solution, followed by concentration, which afforded 531.4 g of a mixture containing 157.1 g (0.625 mol, 97.7% yield) of 2-(2,5-dimethylphenoxymethyl)phenylacetonitrile and 35.4 g (0.102 mol, 99.5% recovery) of 1,2-bis(2,5-dimethylphenoxymethyl)benzene.

### Reference Example 2-(2)

In accordance with Reference Example 1-(1), 372 g of a mixture containing 292 g (1.12 mol, 69.9% yield) of 2-(2,5-dimethylphenoxymethyl)benzyl chloride and 64.3 g (0.186 mol, 23.2% yield) of 1,2-bis(2,5-dimethylphenoxymethyl)benzene was obtained.

A mixture comprising 241 g of water, 60.2 g (1.23 mol) of sodium cyanide, 16.4 g (0.051 mol) of tetra-n-butylammonium bromide and 670 g of toluene was heated to 80°C with stirring, to which a mixture obtained by adding 84.5 g of toluene to 338 g of the mixture produced above was added dropwise over 5 hours, and the mixture was further kept at the same temperature for 2.75 hours.

After cooling to room temperature, the aqueous layer was removed by phase separation, and the organic layer was washed with 345 g of 1% aqueous sodium hydroxide solution and washed twice with 345 g of water, followed by concentration, which afforded 306 g of a mixture containing 248 g (0.987 mol, 97.1% yield) of 2-(2,5-dimethylphenoxymethyl)phenylacetonitrile and 57.5 g (0.166 mol, 98.3% recovery) of 1,2-bis(2,5-dimethylphenoxymethyl)benzene.

### Reference Example 2-(3)

A mixture comprising 196 g of water, 324 g of toluene, 49.0 g (1.00 mol) of sodium cyanide and 12.9 g (0.040 mol) of tetra-n-butylammonium bromide was heated to 80°C with stirring, to which a mixture obtained by adding 313 g of toluene to 313 g of the solution produced in Reference Example 1-(5) was added dropwise over 5 hours, and the mixture was further kept at the same temperature for 3 hours.

After cooling to room temperature, the aqueous layer was removed by phase separation, and the organic layer was washed three times with 338 g of 5% aqueous sodium hydroxide solution, and washed with 338 g of water and then with 338 g of 10% aqueous sodium chloride solution, followed by concentration, which afforded 725 g of a mixture containing 202 g (0.745 mol, 93.2% yield) of 2-(4-chloro-2-methylphenoxymethyl)phenylacetonitrile and 66.2 g (0.171 mol, 99.4% recovery) of 1,2-bis(4-chloro-2-methylphenoxymethyl)benzene.

### Reference Example 3-(1) Production of α-hydroxyiminophenylacetonitrile

A mixture comprising 500 g of water, 434 g of toluene, 103.5 g (1.50 mol) of sodium nitrite and 113.4 g (1.53 mol) of n-butanol was cooled to 0°C with stirring, to which 156.3 g (1.50 mol) of 35% hydrochloric acid was added dropwise over 5 hours, and the mixture was further kept at the same temperature for 2 hours. This mixture was subjected to phase separation, and the organic layer was washed twice with 250 g of 4% sodium hydrogencarbonate and further washed with 250 g of 20% saline solution.

The toluene solution of butyl nitrite thus obtained was analyzed by gas chromatography with an internal standard method. As a result, the content of butyl nitrite was 26.1% and the yield was 98.7% on the basis of sodium nitrite.

To 527.2 g of a toluene solution of a mixture containing 155.8 g (0.620 mol) of 2-(2,5-dimethylphenoxymethyl)phenylacetonitrile and 35.1 g (0.101 mol) of 1,2-bis(2,5-dimethylphenoxymethyl)benzene, which had been obtained in accordance with Reference Example 2-(1), were added 50.3 g (0.896 mol) of potassium hydroxide and 125.7 g of n-butanol with stirring at 22-25°C, to which 308.3 g of a toluene solution containing 79.2 g (0.768 mol) of butyl nitrite produced in accordance with the foregoing was added dropwise at the same temperature over 5 hours, and the mixture was further kept at the same temperature for 2 hours.

To this reaction mixture was added 620 g of water, and the mixture was heated to 60°C and further kept at the same temperature for 3 hours. This mixture was heated under reduced pressure, and toluene and n-butanol were removed by distillation while adding water followed by two washings with 300 g of toluene.

Thus, 1262.7 g of an aqueous solution containing 189.1 g (0.594 mol, 95.8% yield, E/Z = 15/85) of the potassium salt of α-hydroxyimino-2-(2,5-dimethylphenoxymethyl)phenylacetonitrile was obtained. It was found that 1,2-bis(2,5-dimethylphenoxymethyl)benzene was not found in the aqueous solution, but contained at an amount of 33.7 g (0.097 mol, 96.1% recovery) in 594 g of toluene after washing.

### Reference Example 3-(2)

To 50.42 g of toluene were added 29.77 g of the mixture obtained in Reference Example 2-(2), 6.46 g of methanol and 6.46 g (115 mmol) of potassium hydroxide with stirring at room temperature, to which 11.87 g (115 mmol) of commercially available butyl nitrite was added dropwise at the same temperature over 2 hours, and the mixture was further stirred at the same temperature for 3 hours. Then, 104 g of the homogeneous solution obtained was divided into two portions.

To 52 g of one of the two-divided solutions was added 50 g of water, followed by extraction and phase separation, and the aqueous layer obtained was washed twice with 25 ml of toluene, cooled to 15°C and adjusted to pH 1 by use of 36% hydrochloric acid. Then, 50 ml of diethyl ether was added for extraction, followed by phase separation, and the aqueous layer was further extracted twice with 25 ml of diethyl ether. All the ether layers obtained were combined together, and these layers were washed three times with 30 ml of 10% saline solution, dried with anhydrous sodium sulfate and concentrated, which afforded 13.65 g of a pale pink solid.

The analysis by high performance liquid chromatography revealed that the content of α-hydroxyimino-2-(2,5-dimethylphenoxymethyl)phenylacetonitrile was 95.1% (96.5% yield, E/Z = 20/80), but 1,2-bis(2,5-dimethylphenoxymethyl)benzene was not contained.

### Reference Example 3-(3)

First, 52 g of the other of the two-divided solution in Reference Example 3-(2) was cooled to 15°C and adjusted to pH 1 by use of 36% hydrochloric acid, to which 50 ml of diethyl ether and 25 ml of water were added, followed by extraction and phase separation. The aqueous layer was further extracted twice with 25 ml of diethyl ether, and all the separated ether layers were combined together, washed three times with 30 ml of 10% saline solution, dried with anhydrous sodium sulfate and concentrated, which afforded 17.65 g of a brown solid.

The content of α-hydroxyimino-2-(2,5-dimethylphenoxymethyl)phenylacetonitrile was 74.5% (97.7% yield, E/Z = 20/80).

The content of 1,2-bis(2,5-dimethylphenoxymethyl)benzene was 15%.

### Reference Example 3-(4)

To 264.5 g of a toluene solution of a mixture containing 75.40 g (300 mmol) of 2-(2,5-dimethylphenoxymethyl)phenylacetonitrile and 17.54 g (50.6 mmol) of 1,2-bis(2,5-dimethylphenoxymethyl)benzene, which had been obtained in accordance with Reference Example 2-(2), were added 23.57 g (420 mmol) of potassium hydroxide and 58.93 g of n-butanol with stirring at room temperature, to which 142.1 g of a toluene solution containing 37.12 g (360 mmol) of butyl nitrite produced in accordance with Reference Example 3-(1) was added dropwise at the same temperature over 5 hours, and the mixture was further kept at the same temperature for 2.8 hours.

To 486.9 g of the homogeneous solution obtained was added 298 g of water, and the mixture was heated to 50°C and further kept at the same temperature for 3 hours. This mixture was heated under reduced pressure, and toluene and n-butanol were removed by distillation while adding water to yield 635 g, followed by two washings with 150g of toluene.

Thus, 624.9 g of an aqueous solution containing 91.15 g (286 mmol, 95.4% yield, E/Z = 18/82) of the potassium salt of α-hydroxyimino-2-(2,5-dimethylphenoxymethyl)phenylacetonitrile was obtained, which was neutralized with 36% hydrochloric acid. It was found that 1,2-bis(2,5-dimethylphenoxymethyl)benzene was not found in the aqueous solution, but contained at an amount of 17.43 g (50.3 mmol, 99.4% recovery) in 299 g of the toluene layer after washing.

### Reference Example 3-(5)

To 676.1 g of a toluene solution of a mixture containing 188.9 g (695 mmol) of 2-(4-chloro-2-methylphenoxymethyl)phenylacetonitrile and 61.8 g (160 mmol) of 1,2-bis(4-chloro-2-methylphenoxymethyl)benzene, which had been produced in Reference Example 2-(3), were added 56.8 g (978 mmol) of potassium hydroxide and 142 g of n-butanol with stirring at room temperature, to which 86.47 g (839 mmol) of butyl nitrite was added dropwise at the same temperature over 5 hours, and the mixture was further kept at the same temperature for 3 hours.

To 961 g of the homogeneous solution obtained was added 588 g of water, and the mixture was heated under reduced pressure, and toluene and n-butanol were removed by distillation while adding water to yield 1271 g, followed by two washings with 350 g of toluene.

Thus, 1288 g of an aqueous solution containing 239.7 g (707 mmol, 102% yield) of the potassium salt of α-hydroxyimino-2-(4-chloro-2-methylphenoxymethyl)phenylacetonitrile was obtained, which was neutralized with 36% hydrochloric acid. It was found that 1,2-bis(4-chloro-2-methylphenoxymethyl)benzene was not found in the aqueous solution, but contained at an amount of 61.8 g (160 mmol, 100% recovery) in 683 g of the toluene layer after washing.

### Example 1

To a mixture of 185 g of toluene and 3.70 g (0.0115 mol) of tetra-n-butylammonium bromide were simultaneously added dropwise 34.8 g (0.276 mol) of dimethyl sulfate and 461 g of an aqueous solution containing 69.6 g (0.219 mol) of the potassium salt of α-hydroxyimino-2-(2,5-dimethylphenoxymethyl)phenylacetonitrile over 5 hours while keeping at 20 to 25°C, and the reaction was subsequently allowed to proceed at the same temperature for 1 hour. The aqueous layer was removed from the reaction mixture by phase separation, and the organic layer was washed with 65 g of 5% aqueous sodium hydroxide solution and then washed twice with 65 g of 10% saline solution, which afforded 392 g of a toluene solution containing α-methoxyimino-2-(2,5-dimethylphenoxymethyl)phenylacetonitrile. The toluene solution obtained was analyzed by high performance liquid chromatography, and it was found that the yield of α-methoxyimino-2-(2,5-dimethylphenoxymethyl)phenylacetonitrile was 96.5%, and the yield of the nitrone compound as a by-product was 3.4%.

Then, 10.82 g of the above toluene solution containing α-methoxyimino-2-(2,5-dimethylphenoxymethyl)phenylacetonitrile was concentrated under reduced pressure and dried, which afforded 1.91 g of α-methoxyimino-2-(2,5-dimethylphenoxymethyl)phenylacetonitrile. The analysis by high performance liquid chromatography revealed that the content of α-methoxyimino-2-(2,5-dimethylphenoxymethyl)phenylacetonitrile was 88.9%.

### Example 2

To a mixture of 17 g of toluene, 20 g of water, 1.39 g (24.0 mmol) of potassium hydroxide, 5.94 g (20.0 mmol, E/Z = 20/80) of α-hydroxyimino-2-(2,5-dimethylphenoxymethyl)phenylacetonitrile produced in accordance with Reference Example 3-(2) and 0.32 g (1.00 mmol) of tetra-n-butylammonium bromide was added dropwise 3.03 g (24.0 mmol) of dimethyl sulfate over 5 hours while keeping at 20-25°C, and the reaction was subsequently allowed to proceed by keeping at the same temperature for 1 hour. The aqueous layer was removed from the reaction mixture by phase separation, and the organic layer was washed twice with water and then concentrated, which afforded 6.17 g of a solid containing α-methoxyimino-2-(2,5-dimethylphenoxymethyl)phenylacetonitrile. The analysis by high performance liquid chromatography revealed that the yield of α-methoxyimino-2-(2,5-dimethylphenoxymethyl)phenylacetonitrile was 81.3% (E/Z = 8/92), and the yield of the nitrone compound as a by-product was 15.6% (E/Z = 81/19).

### Comparative Example 1

To a mixture of 17 g of toluene, 20 g of water, 1.39 g (24.0 mmol) of potassium hydroxide and 5.94 g (20.0 mmol, E/Z = 20/80) of α-hydroxyimino-2-(2,5-dimethylphenoxymethyl)phenylacetonitrile produced in accordance with Reference Example 3-(2) was added dropwise 3.03 g (24.0 mmol) of dimethyl sulfate over 5 hours while keeping at 20-25°C, and the reaction was subsequently allowed to proceed by keeping at the same temperature for 1 hour. The aqueous layer was removed from the reaction mixture by phase separation, and the organic layer was washed twice with water and then concentrated, which afforded 6.08 g of a solid containing α-methoxyimino-2-(2,5-dimethylphenoxymethyl)phenylacetonitrile. The analysis by high performance liquid chromatography revealed that the yield of α-methoxyimino-2-(2,5-dimethylphenoxymethyl)phenylacetonitrile was 71.7% (E/Z = 6/94), and the yield of the nitrone compound as a by-product was 23.9% (E/Z = 64/36).

### Example 3

To a mixture of 101 g of toluene and 0.91 g (2.8 mmol) of tetra-n-butylammonium bromide were simultaneously added dropwise 8.57 g (67.9 mmol) of dimethyl sulfate and 111 g of an aqueous solution containing 17.7 g (52.3 mmol) of the potassium salt of α-hydroxyimino-2-(4-chloro-2-methylphenoxymethyl)phenylacetonitrile produced in accordance with Reference Example 3-(5) over 3 hours while keeping at 20-25°C, and the reaction was subsequently allowed to proceed at the same temperature for 2 hour. The aqueous layer was removed from the reaction mixture by phase separation, and the organic layer was washed with 47 g of 5% aqueous sodium hydroxide solution and washed twice with 47 g of 10% saline solution, which afforded 249 g of a toluene solution containing α-methoxyimino-2-(4-chloro-2-methylphenoxymethyl)phenylacetonitrile. The toluene solution obtained was analyzed by high performance liquid chromatography, and it was found that the yield of α-methoxyimino-2-(4-chloro-2-methylphenoxymethyl)phenylacetonitrile was 98.5%.

### Example 4

To a mixture of 977 g of toluene and 9.44 g (0.029 mol) of tetra-n-butylammonium bromide were simultaneously added dropwise 84.3 g (0.668 mol) of dimethyl sulfate and 1267.6 g of an aqueous solution containing 177.2 g (0.557 mol, E/Z = 15/85) of the potassium salt of α-hydroxyimino-2-(2,5-dimethylphenoxymethyl)phenylacetonitrile produced in accordance with Reference Example 3-(1) over 4.8 hours while keeping at 23-25°C, and the reaction was subsequently allowed to proceed at the same temperature for 2.5 hour. The aqueous layer was removed from the reaction mixture by phase separation, and the organic layer was washed with 489 g of 5% aqueous sodium hydroxide solution and then with 176 g of 10% saline solution, which afforded 1182.7 g of a toluene solution containing α-methoxyimino-2-(2,5-dimethylphenoxymethyl)phenylacetonitrile. (The hue in 5-fold dilution was No. 16 on the Gardner scale.)

To 1162.7 g of the toluene solution was added dropwise 37.0 g (0.055 mol) of 11% aqueous sodium hypochlorite solution at 26°C, and stirring was continued at the same temperature for 3 hours. To the reaction mixture was added 138 g of 5% aqueous sodium sulfite solution, and the organic layer was washed with 400 g of 5% saline solution and then with 200 g of 10% saline solution, after which the slightly precipitated solid was removed by filtration. The filtrate was allowed to stand and subjected to phase separation to remove the aqueous layer, and the organic layer was concentrated, which afforded a 1169.9 g of a toluene solution containing α-methoxyimino-2-(2,5-dimethylphenoxymethyl)phenylacetonitrile. (The hue in 5-fold dilution was No. 10 on the Gardner scale.)

The toluene solution obtained was analyzed by high performance liquid chromatography, and it was found that the yield of α-methoxyimino-2-(2,5-dimethylphenoxymethyl)phenylacetonitrile was 98.5% (E/Z = 17/83).

### Example 5

To a mixture of 1184 g of toluene and 17.3 g (0.054 mol) of tetra-n-butylammonium bromide were simultaneously added dropwise 163 g (1.29 mol) of dimethyl sulfate and 1866 g of an aqueous solution containing 344 g (1.08 mol, E/Z = 16/84) of the potassium salt of α-hydroxyimino-2-(2,5-dimethylphenoxymethyl)phenylacetonitrile produced in accordance with Reference Example 3-(2) over 5 hours while keeping at 20-25°C, and the reaction was subsequently allowed to proceed at the same temperature for 1 hour. The aqueous layer was removed from the reaction mixture by phase separation, and the organic layer was washed with 5% aqueous sodium hydroxide solution and then washed twice with 296 g of 10% saline solution, which afforded 1841 g of a toluene solution containing α-methoxyimino-2-(2,5-dimethylphenoxymethyl)phenylacetonitrile. The toluene solution obtained was analyzed by high performance liquid chromatography, and it was found that the yield of α-methoxyimino-2-(2,5-dimethylphenoxymethyl)phenylacetonitrile was 91.6% (E/Z = 18/82) and the yield of the nitrone compound as a by-product was 5.7% (E/Z = 91/9).

### Example 6

To a mixture of 924 g of toluene and 13.6 g (0.042 mol) of tetra-n-butylammonium bromide were simultaneously added dropwise 127 g (1.01 mol) of dimethyl sulfate and 1460 g of an aqueous solution containing 268 g (0.84 mol, E/Z = 19/81) of the potassium salt of α-hydroxyimino-2-(2,5-dimethylphenoxymethyl)phenylacetonitrile produced in accordance with Reference Example 3-(2) over 5 hours while keeping at 20-25°C, and the reaction was subsequently allowed to proceed at the same temperature for 1.5 hours. The aqueous layer was removed from the reaction mixture by phase separation, and the organic layer was washed with 1000 g of 5% aqueous sodium hydroxide solution and then washed twice with 250 g of 10% saline solution. The organic layer was concentrated under reduced pressure and dried, which afforded 245 g of α-methoxyimino-2-(2,5-dimethylphenoxymethyl)phenylacetonitrile. The analysis by high performance liquid chromatography revealed that the content and yield of α-methoxyimino-2-(2,5-dimethylphenoxymethyl)phenylacetonitrile was 89.6% and 88.6% (E/Z = 17/83), respectively, and the content and yield of the nitrone compound as a by-product was 4.8% and 4.7% yield (E/Z = 91/9), respectively.

### Example 7

First, 1720 g of a toluene solution containing 116 g (367 mmol, E/Z = 21.4/78.6) of α-methoxyimino-2-(4-chloro-2-methylphenoxymethyl)phenylacetonitrile produced in accordance with Example 3 was subjected to solvent removal, followed by drying under reduced pressure, which afforded 123 g of a brown powder. To this powder was added 262 g of toluene for dissolution, and recrystallization gave 77.2 g of a white powder (66.8% recovery). This powder was analyzed by high performance liquid chromatography, and it was found that the purity of α-methoxyimino-2-(4-chloro-2-methylphenoxymethyl)phenylacetonitrile was 99.7% (E/Z = 0.3/99.7).

The mother liquid of the recrystallization was concentrated and dried to give 45.53 g of a viscous solid. To 20 g (84.6% purity, E/Z = 63.5/36.5) of this solid were added 48.2 g of toluene and 27.2 g (267 mmol) of 36% hydrochloric acid, followed by heating to 80°C, and the reaction was allowed to proceed at the same temperature for 23 hours. To the reaction mixture obtained was added 30 g of water, and the aqueous layer was removed by phase separation. Two washings with 30 g of water were conducted, and the solvent was removed by distillation under reduced pressure until the total weight became 56.4 g. This residue was analyzed by high performance liquid chromatography, and it was found that the E/Z ratio was changed to 29.0/71.0. This residue was recrystallized to give 8.33 g of a white powder of α-methoxyimino-2-(4-chloro-2-methylphenoxymethyl)phenylacetonitrile (16.3% recovery). This powder was analyzed by high performance liquid chromatography, and it was found that the purity of α-methoxyimino-2-(4-chloro-2-methylphenoxymethyl)phenylacetonitrile was 96.6% (E/Z = 0.8/99.2).

### Reference Example 4 Production of starting materials

### (1) Production of 2-phenoxybenzyl bromide

First, 120.0 g (651 mmol) of 2-phenoxytoluene was dissolved in 240 g of chlorobenzene, and the solution was heated to 135-140°C for reflux. To this solution was introduced 105.0 g (657 mmol) of bromine gas heated to 140-150°C over 4 hours, and the stirring was further continued at 135-140°C for 1.5 hours.

Then, the reaction mixture was allowed to stand for cooling to room temperature, which afforded a chlorobenzene solution (408.1 g) containing 122.9 g (467 mmol, 71.7% yield) of 2-phenoxybenzyl bromide, 16.5 g (89.3 mmol, 13.7% recovery) of 2-phenoxytoluene and 32.0 g (93.6 mmol, 14.4% yield) of 2-phenoxybenzal bromide.

### (2) Production of 2-phenoxyphenylacetonitrile

To the above chlorobenzene solution (393.3 g) was added 7.25 g (22.5 mmol) of tetra-n-butylammonium bromide, and the mixture was heated to 78-82°C, to which 80.9 g (495 mmol) of 30% aqueous sodium cyanide solution was added dropwise over 3 hours, and the stirring was further continued at 78-82°C for 2 hours.

Then, the mixture was allowed to stand for coofing to room temperature, and the reaction mixture separated into two layers was subjected to phase separation to give the organic layer. The organic layer was washed with water and then concentrated. Thus, a mixture containing 92.6 g (442 mmol, 98.3% yield) of 2-phenoxyphenylacetonitrile, 15.8 g (85.7 mmol, 99.7% recovery) of 2-phenoxytoluene and 30.9 g (90.4 mmol, 100% recovery) of 2-phenoxybenzal bromide was obtained.

### Reference Example 5

### Production example of α-hydroxyimino-2-phenoxyphenylacetonitrile

### (1) Preparation of methyl nitrite

A mixture comprising 35.6 g (516 mmol) of sodium nitrite, 17.9 g (559 mmol) of methanol and 21.5 g of water was gently stirred at room temperature to give a suspension. To this suspension was added dropwise 44.28 g (271 mmol) of 60% sulfuric acid with stirring at room temperature over 8 hours, which gave methyl nitrite (about 516 mmol) in gas form over 8 hours for use in the subsequent reaction.

### (2) Production of α-hydroxyimino-2-phenoxyphenylacetonitrile

First, 21.3 g (516 mmol) of sodium hydroxide was dissolved in 116.3 g of methanol, and the solution was mixed with a chlorobenzene solution (367.1 g) of a mixture containing 90.0 g (430 mmol) of 2-phenoxyphenylacetonitrile, 15.3 g (83.3 mmol) of 2-phenoxytoluene and 30.1 g (87.9 mmol) of 2-phenoxybenzal bromide, which had been produced in Reference Example 4, followed by stirring at room temperature.

To this mixture was introduced 31.5 g (about 516 mmol) of methyl nitrite prepared in paragraph (1) over 8 hours, and the stirring was further continued at room temperature for 15 hours. The reaction mixture was concentrated to remove methanol and other low-boiling contents, after which 300 ml of water and 300 ml of toluene were added thereto, followed by extraction and phase separation. The aqueous layer obtained was further washed twice with 150 ml of toluene.

The organic layers obtained were combined together and then concentrated, which afforded a mixture containing 0.64 g (3 mmol, 0.7% recovery) of 2-phenoxyphenylacetonitrile, 15.3 g (83.3 mmol, 100% recovery) of 2-phenoxytoluene and 22.4 g (65.6 mmol, 74.6% recovery) of 2-phenoxybenzal bromide.

On the other hand, the aqueous layer obtained was cooled at 0-5°C, and the pH of the reaction mixture was adjusted to 2 or lower by adding dropwise 30.3 g (309 mmol) of 97% sulfuric acid.

Then, 400 ml of toluene was added at room temperature, followed by extraction and phase separation, and the aqueous layer was further extracted once with 150 ml of toluene. The organic layers obtained were combined together, and washed with 150 ml of saline solution, followed by concentration, which afforded 93.7 g (393 mmol, 91.4% yield) of α-hydroxyimino-2-phenoxyphenylacetonitrile. The purity of this product was analyzed by high performance liquid chromatography, and found to be 99.5%.

### Reference Example 6

### (1) Distillation of 2-phenoxybenzyl bromide

A chlorobenzene solution (91.68 g) containing 28.97 g (110 mmol) of 2-phenoxybenzyl bromide, 4.68 g (25.4 mmol) of 2-phenoxytoluene and 5.41 g (15.8 mmol) of 2-phenoxybenzal bromide, which had been produced in accordance with Reference Example 4-(1), was distilled under reduced pressure, and the fractions were collected at a boiling point of 133-134°C/0.3 mmHg to give 16.86 g (64.1 mmol, 58.2% distillation yield) of 2-phenoxybenzyl bromide.

The analysis by gas chromatography revealed that the content was 93.8%.

### (2) Distillation of 2-phenoxyphenylacetonitrile

In this section, 440.8 g of a toluene solution containing 130.9 g (626 mmol) of 2-phenoxyphenylacetonitrile, 23.8 g (129 mmol) of 2-phenoxytoluene and 41 g (120 mmol) of 2-phenoxybenzal bromide, which had been produced in accordance with Reference Example 4-(2), was distilled under reduced pressure, and the fractions were collected at a boiling point of 159°C/3 mmHg to give 101.9 g (487 mmol, 77.8% distillation yield) of 2-phenoxyphenylacetonitrile.

The analysis by gas chromatography revealed that the content was 98.2%.

### Industrial Utilizability

According to the present invention, alkoxyimino-containing compounds are produced in a mixed solvent consisting of a hydrocarbon solvent and water in the presence of a phase transfer catalyst, which makes it possible to reduce the formation of nitrone derivatives as by-products and to produce the alkoxyimino-containing compounds with high purity in high yield.

## Claims

1. A process for producing an α-alkoxyiminophenylacetonitrile derivative of the general formula [III]: 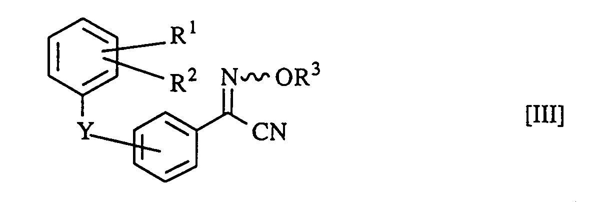 wherein R¹, R², R³ and Y are each as defined below, characterized in that an α-hydroxyiminophenylacetonitrile derivative of the general formula [ I ]: 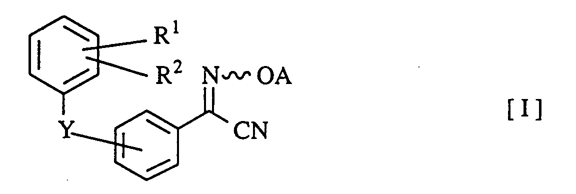 wherein R¹ and R² are the same or different and are independently C₁-C₅ alkyl, C₁-C₅ alkoxy, C₂-C₄ alkenyl, hydrogen, halogen or trifluoromethyl, Y is - O-, -S-, -NCH₃-, -CH₂O-, -OCH₂-, -CH₂S- or -SCH₂-, and A is hydrogen, an alkali metal or an alkaline earth metal, is reacted with a dialkyl sulfate of the general formula [II]:
(R³)₂SO₄ [II]
wherein R³ is C₁-C₅ alkyl, in a mixed solvent consisting of a hydrocarbon solvent and water in the presence of a phase transfer catalyst.

2. A production process according to claim 1, wherein the hydrocarbon solvent is an aromatic solvent.

3. A production process according to claim 2, wherein the aromatic solvent is selected from the group consisting of benzene, toluene, xylene, chlorobenzene and o-dichlorobenzene.

4. A production process according to claim 2, wherein the aromatic solvent is toluene.

5. A production process according to claim 1, wherein the phase transfer catalyst is a quaternary ammonium salt.

6. A production process according to claim 1, wherein the α-hydroxyiminophenylacetonitrile derivative is α-hydroxyimino-2-(2-methylphenoxymethyl)phenylacetonitrile, α-hydroxyimino-2-(2,5-dimethylphenoxymethyl)phenylacetonitrile or α-hydroxyimino-2-(4-chloro-2-methylphenoxymethyl)phenylacetonitrile.

7. A production process according to claim 1, wherein the α-hydroxyiminophenylacetonitrile derivative of the general formula [ I ] is produced by reacting a substituted phenylacetonitrile derivative of the general formula [IV]: 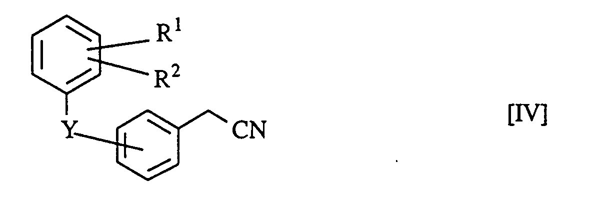 wherein R¹, R² and Y are each as defined above, with an alkyl nitrite of the general formula [V]:
R⁴-ONO [V]
wherein R⁴ is C₁-C₆ alkyl.

8. A production process according to claim 7, wherein the substituted phenylacetonitrile derivative of the general formula [IV] is produced by reacting a benzyl halide derivative of the general formula [VIII]: 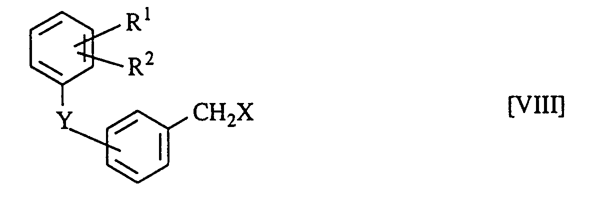 wherein R¹, R², X and Y are each as defined above, with a cyano compound.

9. A production process according to claim 8, wherein the benzyl halide derivative of the general formula [VIII] is produced by reacting a toluene derivative of the general formula [IX]: 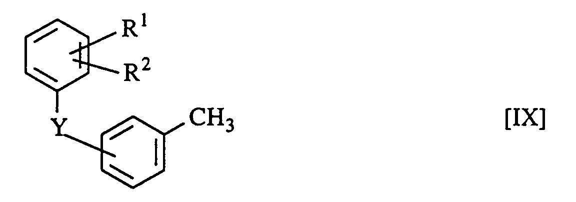 wherein R¹, R² and Y are each as defined above, with halogen.

10. A production process according to claim 7, wherein the Y of the substituted phenylacetonitrile derivative of the general formula [IV] is -OCH₂- or -SCH₂-.

11. A production process according to claim 10, wherein the substituted phenylacetonitrile derivative of the general formula [IV] is produced by reacting a benzyl halide derivative of the general formula [VIII]: 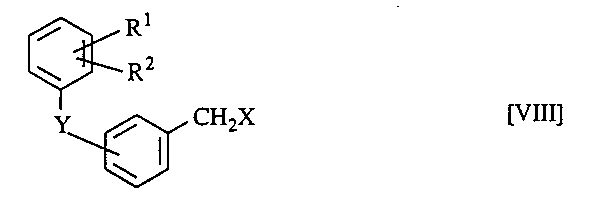 wherein R¹ and R² are each as defined above, Y is -OCH₂- or -SCH₂-, and X is halogen, with a cyano compound.

12. A production process according to claim 11, wherein the benzyl halide derivative of the general formula [VIII] is produced by reacting an α,α'-dihaloxylene derivative of the general formula [VI]: 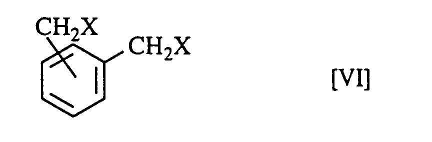 wherein X is as defined above, with a metal salt of a phenol derivative of the general formula [VII]: 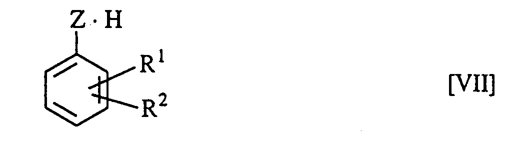 wherein R¹ and R² are each as defined above, and Z is O or S.

## Patentansprüche

1. Verfahren zur Herstellung eines α-Alkoxyiminophenylacetonitrilderivats der allgemeinen Formel [III]: 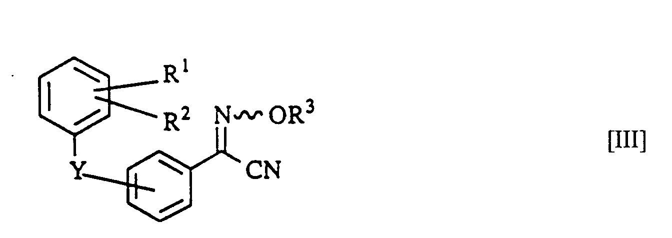 in der R¹, R², R³ und Y jeweils wie nachstehend definiert sind, dadurch gekennzeichnet, daß ein α-Hydroxyiminophenylacetonitrilderivat der allgemeinen Formel [I]: 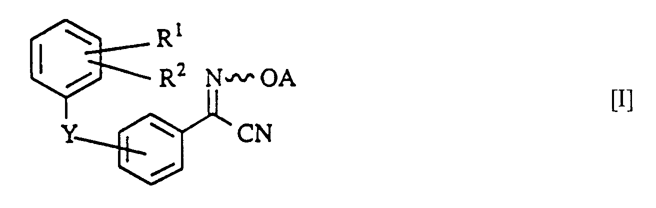 in der R¹ und R² gleich oder verschieden sind und unabhängig voneinander ein C₁-C₅-Alkyl-, C₁-C₅-Alkoxy-, C₂-C₄-Alkenylrest, ein Wasserstoff-, Halogenatom oder eine Trifluormethylgruppe bedeuten, Y eine Gruppe der Formel ―O―, ―S―, ―NCH₃―, ―CH₂O―, ―OCH₂―, ―CH₂S―, ―SCH₂― darstellt, und A ein Wasserstoffatom, Alkalimetall oder Erdalkalimetall bedeutet, mit einem Schwefelsäuredialkylester der allgemeinen Formel [II]:
(R³)₂SO₄ [II]
in der R³ einen C₁-C₅-Alkylrest darstellt, in einem Lösungsmittelgemisch aus einem Kohlenwasserstofflösungsmittel und Wasser in Gegenwart eines Phasentransferkatalysators umgesetzt wird.

2. Herstellungsverfahren nach Anspruch 1, wobei das Kohlenwasserstofflösungsmittel ein aromatisches Lösungsmittel ist.

3. Herstellungsverfahren nach Anspruch 2, wobei das aromatische Lösungsmittel aus Benzol, Toluol, Xylol, Chlorbenzol und o-Dichlorbenzol ausgewählt ist.

4. Herstellungsverfahren nach Anspruch 2, wobei das aromatische Lösungsmittel Toluol ist.

5. Herstellungsverfahren nach Anspruch 1, wobei der Phasentransferkatalysator ein quartäres Ammoniumsalz ist.

6. Herstellungsverfahren nach Anspruch 1, wobei das α-Hydroxyiminophenylacetonitrilderivat α-Hydroxyimino-2-(2-methylphenoxymethyl)phenylacetonitril, α-Hydroxyimino-2-(2,5-dimethylphenoxymethyl)phenylacetonitril oder α-Hydroxyimino-2-(4-chlor-2-methylphenoxymethyl)phenylacetonitril ist.

7. Herstellungsverfahren nach Anspruch 1, wobei das α-Hydroxyiminophenylacetonitrilderivat der allgemeinen Formel [I] durch Umsetzen eines substituierten Phenylacetonitrilderivats der allgemeinen Formel [IV]: 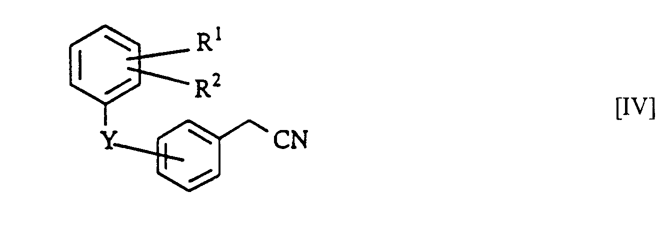 in der R¹, R² und Y jeweils wie vorstehend definiert sind, mit einem Alkylnitrit der allgemeinen Formel [V]:
R⁴―ONO [V]
in der R⁴ einen C₁-C₆-Alkylrest bedeutet, hergestellt wird.

8. Herstellungsverfahren nach Anspruch 7, wobei das substituierte Phenylacetonitrilderivat der allgemeinen Formel [IV] durch Umsetzen eines Benzylhalogenidderivats der allgemeinen Formel [VIII]: 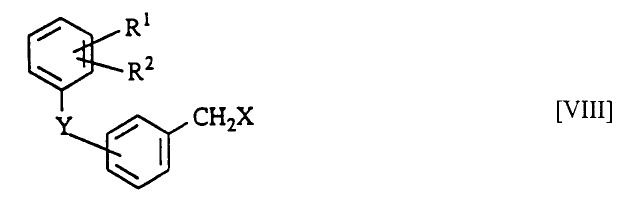 in der R¹, R², X und Y jeweils wie vorstehend definiert sind, mit einer Cyanoverbindung hergestellt wird.

9. Herstellungsverfahren nach Anspruch 8, wobei das Benzylhalogenidderivat der allgemeinen Formel [VIII] durch Umsetzen eines Toluolderivats der allgemeinen Formel [IX]: 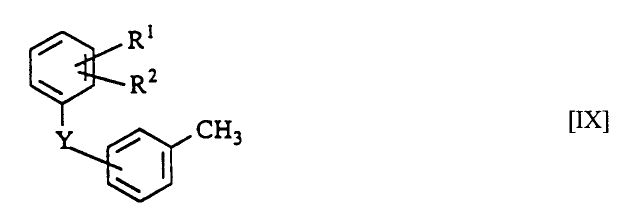 in der R¹, R² und Y jeweils wie vorstehend definiert sind, mit einem Halogen hergestellt wird.

10. Herstellungsverfahren nach Anspruch 7, wobei der Rest Y des substituierten Phenylacetonitrilderivats der allgemeinen Formel [IV] eine Gruppe der Formel ―OCH₂― oder ―SCH₂― bedeutet.

11. Herstellungsverfahren nach Anspruch 10, wobei das substituierte Phenylacetonitrilderivat der allgemeinen Formel [IV] durch Umsetzen eines Benzylhalogenidderivats der allgemeinen Formel [VIII]: 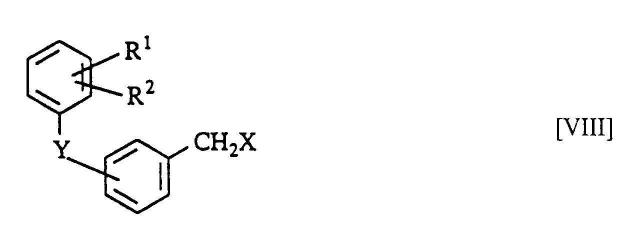 in der R¹ und R² jeweils wie vorstehend definiert sind, Y eine Gruppe der Formel ―OCH₂― oder ―SCH₂― darstellt, und X ein Halogenatom bedeutet, mit einer Cyanoverbindung hergestellt wird.

12. Herstellungsverfahren nach Anspruch 11, wobei das Benzylhalogenidderivat der allgemeinen Formel [VIII] durch Umsetzen eines α,α'-Dihalogenxylolderivats der allgemeinen Formel [VI]: 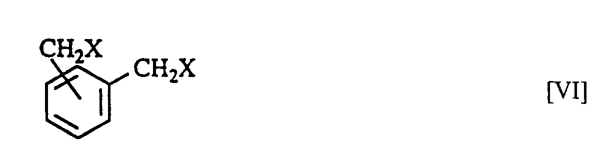 in der X wie vorstehend definiert ist, mit einem Metallsalz eines Phenolderivats der allgemeinen Formel [VII]: 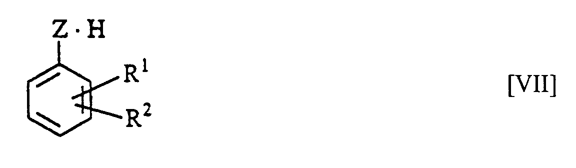 in der R¹ und R² jeweils wie vorstehend definiert sind, und Z ein Sauerstoff- oder Schwefelatom bedeutet, hergestellt wird.

## Revendications

1. Procédé de production d'un dérivé α-alcoxyiminophénylacétonitrile de formule générale [III] : 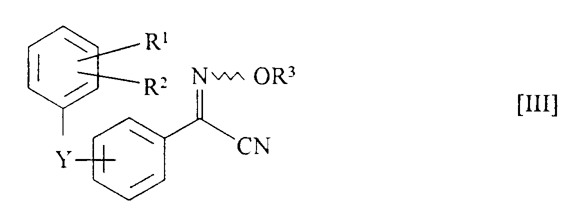 où R¹, R², R³ et Y sont tels que défini ci-dessous, caractérisé en ce qu'un dérivé α-hydroxyiminophénylacétonitrile de formule générale [I] : 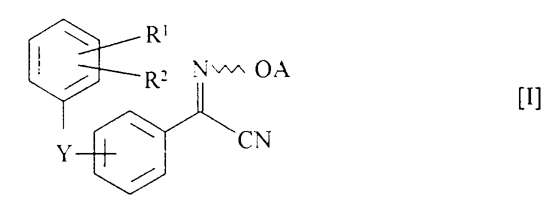 où R¹ et R² sont identiques ou différents et représentent indépendamment un alkyle C₁-C₅, alcoxy C₁-C₅, alcényle C₂-C₄, hydrogène, halogène ou trifluorométhyle, Y est -O-, -S-, -NCH₃-, -CH₂O-, -OCH₂-, -CH₂S- ou -SCH₂- et A est un hydrogène, un métal alcalin ou un métal alcalino-terreux, est mis en réaction avec un sulfate de dialkyle de formule générale [II] :
(R³)₂SO₄ [II]
où R³ est un alkyle C₁-C₅, dans un mélange de solvant constitué d'un solvant hydrocarbure et d'eau en présence d'un catalyseur de transfert de phase.

2. Procédé de production selon la revendication 1 où le solvant hydrocarbure est un solvant aromatique.

3. Procédé de production selon la revendication 2, où le solvant aromatique est choisi dans le groupe constitué de benzène, toluène, xylène, chlorobenzène et o-dichlorobenzène.

4. Procédé de production selon la revendication 2, où le solvant aromatique est le toluène.

5. Procédé de production selon la revendication 1, où le catalyseur de transfert de phase est un sel d'ammonium quaternaire.

6. Procédé de production selon la revendication 1, où le dérivé α-hydroxyiminophénylacétonitrile est le α-hydroxyimino-2-(2-méthylphénoxyméthyl)phénylacétonitrile, α-hydroxyimino-2-(2,5-diméthylphénoxyméthyl)phénylacétonitrile ou α-hydroxyimino-2-(4-chloro-2-méthylphénoxyméthyl)phénylacétonitrile.

7. Procédé de production selon la revendication 1, où le dérivé α-hydroxyiminophénylacétonitrile de formule générale [I] est produit en faisant réagir un dérivé phénylacétonitrile substitué de formule générale [IV] : 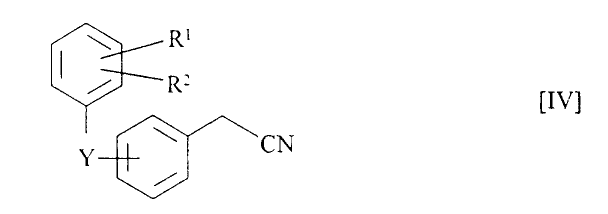 où R¹, R² et Y sont respectivement tels que défini ci-dessus, avec un nitrite d'alkyle de formule générale [V] :
R⁴-ONO [V]
où R⁴ est un alkyle C₁-C₆.

8. Procédé de production selon la revendication 7, où le dérivé phénylacétonitrile de formule générale [IV] est produit en faisant réagir un dérivé halogénure de benzyle de formule générale [VIII] : 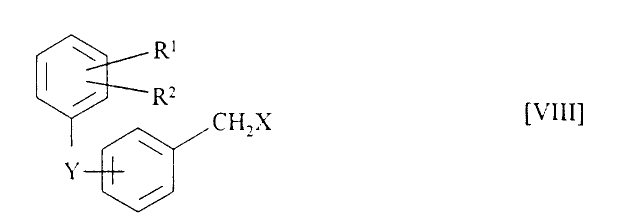 où R¹, R², X et Y sont tels que défini respectivement ci-dessus avec un composé cyano.

9. Procédé de production selon la revendication 8, où le dérivé halogénure de benzyle est dérivé de formule générale [VIII] et produit en faisant réagir un dérivé toluène de formule générale [IX] : 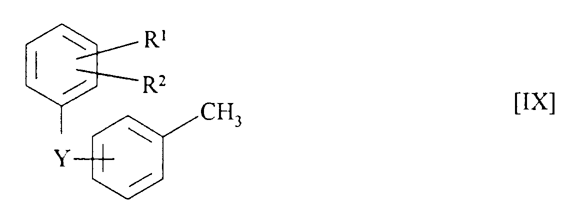 où R¹, R² et Y sont tels que défini ci-dessus, avec un halogène.

10. Procédé de production selon la revendication 7, où le Y du dérivé phénylacétonitrile substitué de formule [IV] est -OCH₂- ou -SCH₂-.

11. Procédé de production selon la revendication 10, où le dérivé phénylacétonitrile substitué de formule générale [IV] est produit en faisant réagir un dérivé halogénure de benzyle de formule générale [VIII] : 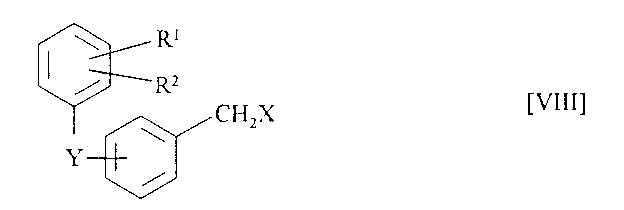 où R¹ et R² sont respectivement définis ci-dessus, Y est -OCH₂- ou -SCH₂-, et X est un halogène, avec un composé cyano.

12. Procédé de production selon la revendication 11, où le dérivé halogénure de benzyle de formule générale [VIII] est produit en faisant réagir un dérivé α,α'-dihaloxylène de formule générale [VI] : 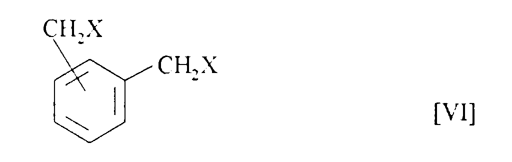 où X est tel que défini ci-dessus, avec un dérivé d'un sel métallique de phénol de formule générale [VII] : 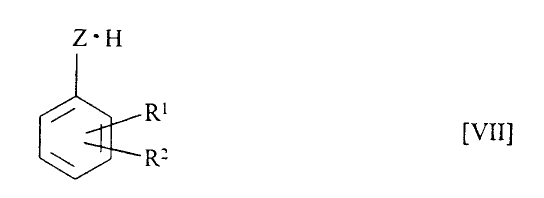 où R¹ et R² sont respectivement tels que défini ci-dessus, et Z est O ou S.
